# NOUVEAU FASCICULE DE BREVET EUROPEEN

(11) **EP 0 465 338 B2**
(45) Date de publication et mention de la décision concernant l'opposition: **26.05.2004**
(45) Mention de la délivrance du brevet: 10.04.1996
(21) Numéro de dépôt: 91401799.1
(22) Date de dépôt: 28.06.1991
(51) Int. Cl.: A61K 9/16, A23K 1/00

(54) **Procédé de préparation de granulés de principes actifs par extrusion**
Verfahren zur Herstellung von Wirkstoffgranulaten durch Extrudierung
Process for the preparation of granulates of actives through extrusion

(30) Priorité: 29.06.1990 FR 9008280
(43) Date de publication de la demande: 08.01.1992
(73) Titulaire: Aventis Animal Nutrition S.A., 67300 Schiltigheim (FR)
(72) Inventeur: Autant, Pierre, F-03600 Commentry (FR); Ruel, Jacques, F-95210 Saint Gratien (FR); McCombs, Charles Allan, Kingsport, Tennessee 37663 (US); Hoskins, Louis Pasteur, Kingsport, Tennessee 37660 (US); Smith, Ernest Phillip, Blountville, Tennessee 37617 (US); Weinhold, Stephen, Kingsport, Tennessee 37664 (US); Wu, Stephen Hong-Wei, Kingsport, Tennessee 37663 (US)
(74) Mandataire: Guerre, Dominique

(56) Documents cités:
- EP-A- 0 204 596
- EP-A- 0 208 144
- EP-A- 0 240 904
- EP-A- 0 276 781
- EP-A- 0 423 000
- GB-A- 2 160 096
- US-A- 4 181 710

## Description

La présente invention concerne un procédé de préparation de granulés de principes actifs. Elle concerne plus particulièrement un procédé de préparation de granulés de principes actifs destinés à l'alimentation ou au traitement des ruminants.

Il est connu par exemple selon les brevets US 4 181 708, 4 181 709 et 4 181 710 de préparer des granulés adaptés à l'administration chez les ruminants composés d'un coeur de substance active et d'un enrobage à base d'une substance hydrophobe et d'un polymère résistant au pH neutre du rumen et dégradable au pH plus acide de la caillette.

Le coeur de ces granulés, composé du principe actif se présente sous forme granulaire. Il est obtenu par mélange à sec des matières actives, d'un ou plusieurs agents liants avec éventuellement des agents de neutralisation, mélange qui est ensuite mouillé par addition de 10 à 20 % d'eau, transformé en une masse pâteuse qui par extrusion à température ambiante et sphéronisation est granulée. Les granulés humides sont séchés dans un four ou un lit fluidisé.

Lorsque l'on met en oeuvre la masse humide telle que décrite dans les brevets préalablement mentionnés les granulés obtenus après extrusion et sphéronisation ne se présentent jamais, après séchage, une forme parfaitement sphérique mais présentent toujours des aspérités, ou irrégularités de surface et une porosité élevée qui rendent plus contraignantes l'opération ultérieure d'enrobage.

Cette opération d'enrobage est réalisée, encore selon les brevets précités, directement sur les granulés obtenus après séchage par le mélange de polymère et de substances hydrophobes, Ce mélange d'enrobage est mis en solution dans un solvant organique puis pulvérisé sur les granulés.

Pour obtenir une protection correcte du principe actif, il est nécessaire de pulvériser au moins 25 g du mélange polymère et acide gras pour 100 g de matière active granulée et la couche d'enrobage a une épaisseur d'environ 150 µm (voir exemple 1 du brevet US 4 181 710).

Il est connu selon le brevet EP 276781 de préparer des granulés pour ruminants par une méthode qui consiste à granuler par agitation la matière active en présence d'un agent liant ayant un point de fusion compris entre 40 et 120°C, puis à ajouter après formation d'un filmn de substance fusible à la surface du granulé des particules solides ayant une dimension moyenne inférieure à 10 µm. Aucune ètape de pulvérisation de l'enrobant n'est décrité.

Il est encore connu selon le brevet GB 2160096 de préparer des granulés pour ruminants, dans un mélangeur à bandes, contenant un principe actif, un liant et un agent de libération. La quantité d'agent liant et d'agent de libération est toujours supérieure à la quantité de principe actif ce qui ne permet de fabriquer que des granulés contenant moins de 55% de principe actif. Aucune étape d'enrobage per pulvérisation n'est décrite.

La présente invention a cherché à préparer des granulés de principes actifs destinés aux ruminants. Cet objectif a été atteint en utilisant comme coeur de principe actif devant subir l'enrobage, un granulé ayant été, préparé par extrusion d'un mélange contenant un liant à l'état fondu. Ce coeur de principes actifs est obtenu par extrusion d'un mélange contenant les principes actifs, de préférence à l'état solide et un liant fusible en quantité suffisante pour permettre le passage à travers une filière. Cette quantité est de préférence inférieure à 25 % en poids de la masse du granulé.

L'objet de l'invention est un procédé de préparation de granulés de principes actifs alimentaires ou thérapeutiques destinés aux ruminants caractérisé en ce que dans une première étape on prépare les coeurs desdits granulés, par extrusion d'un mélange contenant de 95 à 80% en poids de principes actifs en présence d'un agent liant fusible en quantité telle qu'il permet le passage de la masse dans la filière et d'un polymère, les dits coeurs de granulés présentant après sphéronisation et tamisage une densité supérieure à 1,2 g/ml et une répartition de granulés comprise entre 200 et 4000 microns et dans une deuxième étape on enrobe les dits coeurs par pulvérisation d'une couche d'enrobage protectrice vis-à-vis du rumen.

On préfère utiliser comme liant fusible une cire de polyéthylène, une paraffine, une huile ou une graisse animale, éventuellement hydrogénée, un acide gras contenant 10 à 32 atomes de carbone, les esters ou les alcools correspondants et tout particulièremnet l'acide stéarique. En effet ce dernier présente l'avantage d'être un produit alimentaire et un des constituants préférés de la couche d'enrobage.

Afin d'éviter l'exsudation du liant, lors de l'extrusion, il est souvent avantageux d'ajouter dans la masse un polymère, de préférence soluble dans le liant, tel que les polymères à base de vinyl pyridine et de styrène, d'alkyl cellulose (éthylcellulose) d'hydroxy alkyl cellulose (hydroxy propyl cellulose), de polyvinyl pyrrolidone, les polyéthylènes. On préfère tout particulièrement utiliser le copolymère à base de vinyl pyridine et de styrène qui sert aussi pour la couche d'enrobage ou l'éthylcellulose.

Pour augmenter la densité du granulé il est parfois avantageux d'ajouter une charge choisie parmi les charges minérales (silice, talc).

La composition pondérale préférée pour former le coeur des granulés est la suivante :

| | |
|---|---|
| principes actifs | 95 à 80 % |
| liant fusible | 5 à 20 % |
| polymère inférieur ou égal à | 2 %. |

Une composition encore plus préférentielle est constituée de :

| | |
|---|---|
| principes actifs 90 à | 84 % |
| liant fusible 10 à | 16 % |
| polymère inférieur ou égal à | 1 %. |

Les granulés obtenus par le procédé de l'invention se présentent sous une forme matricielle, c'est à dire que les particules de principes actifs sont assemblées entre elles par l'intermédiaire du liant ces particules se trouvent ainsi dans un état pendulaire, funiculaire et à la limite d'un état capillaire (Granulation Sherrington & R. Oliver, Heyden & Son, p.7-18, 1981).

Selon une méthode de mise en oeuvre, la masse à extruder, après chauffage au dessus de la température de fusion du liant, est forcée à travers une extrudeuse munie d'une ou plusieurs filières présentant le diamètre du granulé final désiré. Les extrudats à la sortie de la filière sont éventuellement coupés au moyen d'un couteau rotatif.

L'extrusion par voie fondue, présente l'avantage par rapport aux procédés de l'art antérieur d'éviter la solubilité au moins partielle du principe actif dans l'eau, solubilité qui a tendance à modifier le comportement de la masse au cours de l'extrusion. La sphéronisation des granulés obtenus par le procédé selon l'invention est aussi facilitée car la surface du granulé présente des qualités de plasticité dues à la fusion superficiel le du liant qui n'existaient pas dans les granulés obtenus par voie humide. Les granulés obtenus, après sphéronisation présentent une surface régulière, lisse et exigent ainsi une quantité de polymère d'enrobage plus réduite pour une efficacité équivalente.

En comparaison avec l'art antérieur qui utilise des procédés en voie humide, le procédé de la présente invention présente encore l'avantage d'éviter les opérations de séchage qui diminuent la densité du granulé (1,0 à 1,1 g/ml contre 1,2 g/ml pour les granulés de la présente invention) par formation de microporosités. L'augmentation de densité est importante car il a été démontré que pour éviter les pertes de principes actifs à la mastication il était avantageux d'avoir une densité minimale de 1,2.

Le procédé par voie humide était aussi très sensible à l'opération de mélange préalable à l'extrusion, en particulier à la température et au temps de mélange, alors que le procédé selon l'invention par voie fondue ne présente pas cet inconvénient.

Les principes actifs sont choisis parmi les amino acides tels que la méthionine, la lysine ou ses sels, la phénylalanine, l'histidine, l'arginine, la tyrosine, le tryptophane, les médicaments tels que les vitamines, les antibiotiques, les antiparasitaires, les protéines.

Les granulés obtenus après un éventuel traitement de sphéronisation, sont tamisés de façon à conserver une répartition de granulés comprise entre 200 et 4000 µm et de préférence entre 500 µm et 2500 µm.

La couche d'enrobage, protectrice vis à vis du rumen, est ensuite pulvérisée selon la technique décrite dans les brevets américains précités.

L'enrobage contient au moins un élément qui est choisi parmi les polymères, les copolymères ou les mélanges basiques dont le taux d'azote est compris entre 2 et 14 % et le poids moléculaire est compris entre 50 000 et 500 000.

Pour la définition des polymères et copolymères on se reportera à leur définition colonne 7 du brevet US 4181 710.

On préfère parmi les copolymères utiliser le copolymère styrène vinyl-2 pyridine (contenant 50 à 80 % en poids de vinyl-2 pyridine et 20 % à 50 % de styrène).

L'enrobant contient aussi une substance hydrophobe choisie parmi les acides gras ayant 12 à 32 atomes de carbone. Ils sont aussi décrits dans le brevet US 4 181 710.

On préfère parmi les substances hydrophobes utiliser l'acide stéarique.

L'enrobant préféré selon l'invention a la composition pondérale suivante :
10-30 % de copolymère vinyl-2 pyridine - styrène
70-90 % d'acide stéarique.

Le mélange enrobant contenant le copolymère et la substance hydrophobe est mis en solution dans un solvant halogéné, un alcool, un éther, une cétone ou un mélange de ces solvants. Il est tout particulièrement avantageux d'utiliser un mélange éthanol/dichloro-1,2 éthane, éthanol/chlorure de méthylène, éthanol/acétone.

La solution de mélange enrobant est pulvérisée sur les coeurs de principes actifs, obtenus par le procédé de la présente invention, à l'aide d'un lit fluidisé ou de tout autre appareil de pulvérisation. On préfère utiliser, pour la pulvérisation, un appareil connu sous la dénomination de spray-coating, par exemple de type UNIGLATT® équipé d'une cuve WURSTER®.

La quantité d'enrobant utilisée, exprimée en matière sèche par rapport au coeur granulé est comprise notamment entre 10 et 30 % et de préférence entre 15 et 25 % ce qui pour des granulés ayant un diamètre moyen compris entre 500 µm et 1 600 µm représente une épaisseur d'enrobage comprise entre 20 et 70 µm.

La présente ivention sera plus complètement décrite à l'aide des exemples suivants.

### EXEMPLE 1

### 1) Matériel utilisé

mélangeur planétaire DITO SAMA®
extrudeuse monovis WYSS®, diamètre 35 mm, PHARMEX 35 T®
alimentation de l'extrudeuse par vis K TRON
couteau granulateur à vitesse variable à quatre lames souples qui frotte contre la plaque filière et dont l'axe est dans l'axe de la vis
sphéroniseur WYSS® diamètre 300 mm, SPHAEROMAT 300®

### 2) Conditions opératoires

| | | |
|---|---|---|
| **extrusion:** | température de la double enveloppe | 130°C |
| | vitesse de rotation de la vis | 150 t/mn |
| | lilière: diamètre de l'orifice | 1,5 mm |
| | angle chanfrein d'entrée | 60° |
| | longueur du canal cylindrique | 3,5 mm |
| | épaisseur totale | 5 mm |
| | nombre d'orifices | 84 |

**alimentation**: débit de la vis K-Tron réglée au débit d'extrusion

| | | |
|---|---|---|
| **coupe:** | couteau rotatif axial à quatre lames vitesse de rotation | 500 t/mn |
| **sphéronisation:** | température de la double enveloppe | 120°C |
| | vitesse de rotation du plateau | 1150 t/mn |

### 3) Préparation des mélanges

Le liant est composé d'acide stéarique pur (UNICHEMA® Prifrac 2981) et d'éthylcellulose (DOW 100®). Les mélanges initiaux sont préparés par agitation pendant 15 minutes à température ambiante par charge de un kilo dans un mélangeur planétaire (DITO SAMA®). Les différents constituants, méthionine et liant, sont introduits séparément. Le débit moyen d'extrusion est de 6,25 kg/h, la granulométrie du produit après sphéronisation est la suivante:

| | |
|---|---|
| diamètre > 1,6 mm | 1 % |
| 1,6 > diamètre > 1 | 92 % |
| diamètre < 1 mm | 7 % |

### 4) Variation du taux de liant

On fait varier le taux de liant dans le granulé final entre 10 et 11 %, le liant étant composé d'un mélange acide stéarique/éthyl cellulose (94/6). Les résultats sont indiqués dans le tableau I.

### 5) Variation de la composition du liant

On fait varier le taux relatif d'acide stéarique par rapport à l'éthylcellulose en maintenant le taux de liant à 11 % en poids par rapport à la méthionine. Les résultats sont indiqués dans le tableau II.

### 6) Sphéronisation

Des essais de sphéronisation ont été faits sur la méthionine granulée avec 11 % de taux de liant composé de 95 % d'acide stéarique et de 5 % d'éthyl cellulose, les résultats sont indiqués dans le tableau III.

### EXEMPLE 2

On reproduit l'exemple 1 avec le même appareillage ,les mêmes conditions opératoires, le même liant, acide stéarique/éthylcellulose dans des proportions pondérales 94/6 mais en utilisant comme matière active plusieurs mélanges de méthionine et de lysine dans des proportions variables. Les résultats des essais sont indiqués dans le tableau IV.

### EXEMPLE 3

On reproduit l'exemple 1 en changeant la nature de l'agent liant fusible. La nature des compositions ayant fait l'objet de l'extrusion sont indiquées dans le tableau V. Tous les produits obtenus ont subi la spéronisation sans problème.

**TABLEAU I**

| | | | | |
|---|---|---|---|---|
| taux de liant % du granulé | 11 | 10 | 10,5 | 11 |
| débit moyen d'extrusion kg/h | 6,31 | 6,45 | 6,00 | 6,25 |
| granulométrie de coeur | | | | |
| diamètre > 1,6 mm | 0 | 28 | 3 | 1 |
| 1,6 > diamètre > 1 mm | 90 | 41 | 71 | 92 |
| 1 > diamètre | 9 | 31 | 26 | 7 |
| densité | 1,24 | 1,23 | 1,22 | 1,24 |

**TABLEAU II**

| taux de polymère % du liant | 6 | 5 | 4 | 3 | 2 | 1 | 0 |
|---|---|---|---|---|---|---|---|
| débit moyen d'extrusion kg/h | 6,25 | 6,38 | 6,25 | 6,25 | 6,00 | 6,38 | 6,50 |
| granulomètrie du coeur | | | | | | | |
| diamètre > 1,6 mm | 1 | 1 | 71 | 1 | 12 | 4 | 11 |
| 1,6 > diamètre > 1 mm | 92 | 89 | 25 | 86 | 80 | 86 | 82 |
| 1 > diamètre | 7 | 10 | 4 | 13 | 8 | 10 | 7 |
| densité | 1,24 | 1,23 | 1,23 | 1,23 | 1,23 | 1,23 | 1,20 |
| observation | | | exsudation et encrassement de la filière | | | | |

**TABLEAU III**

| Temps de sphéronisation | 5 sec. | 5 min. | 10 min. | 14 min. |
|---|---|---|---|---|
| Granulométrie du coeur | | | | |
| diamètre > 1,6 mm | 1 | 2 | 2 | 3 |
| 1,6 > diamètre > 1 mm | 64 | 78 | 79 | 81 |
| 1 > diamètre | 35 | 20 | 19 | 16 |
| Densité | 1,22 | 1,23 | 1,25 | 1,25 |

**TABLEAU IV**

| | | | |
|---|---|---|---|
| chlorhydrate de lysine (g) | 59 | 60 | 67 |
| méthionine (g) | 25,3 | 25,8 | 17 |
| acide stéarique (g) | 14,7 | 13,3 | 15 |
| éthylcellulose (g) | 0,9 | 0,8 | 0,9 |
| filière en mm | 1,5 | 1 | 1,5 |
| densité réelle | 1,21 | 1,22 | 1,21 |
| granulométrie | | | |
| diamètre > 2 mm | 23 | 1 | 33 |
| 1,6 < diamètre < 2 | 22 | 0 | 16 |
| 1,4 < diamètre < 1,6 | 30 | 7 | 23 |
| 1,25< diamètre < 1,4 | 15 | 36 | 13 |
| 1 < diamètre < 1,25 | 3 | 8 | 3 |
| diamètre < 1 | 7 | 48 | 2 |

**TABLEAU V**

| Constituants | Fraction pondérale | | | |
|---|---|---|---|---|
| Lysine HCl | 63,75 | 63,75 | 63,75 | 63,75 |
| Méthionine | 21,25 | 21,25 | 21,25 | 21,25 |
| Ethyl cellulose | 1,05 | 1,05 | 1,05 | 1,05 |
| Acide stéarique (1) | 8,37 | | | |
| Acide palmitique (1) | 5,58 | | | |
| Mono stéarate de glycérol | | 13,95 | | |
| Di stéarate de glycérol | | | 13,95 | |
| Suif hydrogéné | | | | 13,95 |

## Revendications

1. Procédé de préparation de granulés de principes actifs alimentaires ou thérapeutiques destinés aux ruminants **caractérisé en ce que** dans une première étape on prépare les coeurs desdits granulés, par extrusion d'un mélange contenant de 95 à 80% en poids de principes actifs en présence d'un agent liant fusible en quantité telle qu'il permet le passage de la masse dans la filière et d'un polymère, les dits coeurs de granulés présentant après sphéronisation et tamisage une densité supérieure à 1,2 g/ml et une répartition de granulés comprise entre 200 et 4000 microns et dans une deuxième étape on enrobe les dits coeurs par pulvérisation d'une couche d'enrobage protectrice vis-à-vis du rumen.

2. Procédé de préparation selon la revendication 1 **caractérisé en ce que** la première étape est réalisée selon l'étape 1 de la revendication 1 et dans une deuxième étape on enrobe, avec une quantité d'enrobant, exprimée en matière sèche par rapport aux dits coeurs, comprise entre 10 et 30% en poids les dits coeurs par pulvérisation.

3. Procédé selon les revendications 1 et 2 **caractérisé en ce que** au cours de la première étape les coeurs desdits granulés contiennent 90 à 84% en poids de principes actifs et au cours de la deuxième étape la quantité d'enrobant est comprise entre 15 et 25% en poids.

4. Procédé selon la revendication 1 **caractérisé en ce que** les principes actifs sont choisis parmi les médicaments ou les aminoacides destinés à l'alimentation des ruminants.

5. Procédé selon la revendication 1 ou 2 **caractérisé en ce que** les principes actifs sont choisis parmi la méthionine et le chlorhydrate de lysine.

6. Procédé selon l'une quelconque des revendications précédentes **caractérisé en ce que** l'agent liant fusible est choisl parmi les cires de polyéthylène, les paraffines, les huiles ou les graisses, éventuellemment hydrogénées, les acides gras ayant 10 à 32 atomes de carbone, les esters et les alcools correspondants.

7. Procédé selon la revendication 4 **caractérisé en ce que** ragent fusible est l'acide stéarique.

8. Procédé selon rune quelconque des revendications précédentes **caractérisé en ce que** l'on ajoute une quantité suffisante de polymère pour obtenir une masse permettant un passage correct à travers la filière et ne provoquant pas de phénomènes d'exsudation lors de l'extrusion.

9. Procédé selon la revendication 6 **caractérisé en ce que** le polymère est choisi parmi les copolymères du styrène avec les vinyl pyridine, les alkylcelluloses, les hydroxyalkylcelluloses, les polyvinyl pyrolidone, les polyéthylènes.

10. Procédé selon la revendication 7 **caractérisé en ce que** le polymère est un copolymère à base de styrène et de vinylpyridine.

11. Procédé selon la revendication 7 **caractérisé en ce que** le polymère est l'éthylcellulose.

12. Coeurs de granulés destinés aux ruminants composés d'un mélange matriciel de 95 à 80% d'un ou plusieurs principes actifs, de 5 à 20% d'un liant fusible et contenant un polymère préparés selon l'étape 1 de la revendication 1.

13. Coeurs selon la revendication 12 composés d'un mélange matriciel de 90 à 84% en poids d'un ou plusleurs principes actifs, de 10 à 16% en poids d'un liant fusible et contenant un polymère préparés selon l'étape 1 de la revendication 1.

14. Coeurs de principes actifs devant subir ultérieurement un enrobage présentant une densité supérieure à 1,2. préparés selon l'étape 1 de la revendication 1.

## Patentansprüche

1. Verfahren zum Herstellen eines Granulats mit Nahrungsmitteloder Arzneimittel-Wirkstoffen für Wiederkäuer, **dadurch gekennzeichnet, dass** man in einer ersten Stufe die Kerne des Granulats durch Extrusion einer Mischung, die 95 bis 80 Gew.-% Wirkstoffe enthält, und zwar in Gegenwart einer solchen Menge eines schmelzbaren Bindemittels, die die Passage der Masse in der Düse erlaubt, und eines Polymers herstellt, wobei die Kerne des Granulats nach der Sphäronisation und nach dem Sieben eine Dichte von mehr als 1,2 g/ml und eine Größenverteilung des Granulats zwischen 200 µm und 4000 µm aufweisen, und dass man in einer zweiten Stufe die Kerne durch Pulverisieren mit einer Umhüllungsschicht, die gegenüber dem Pansen schützt, beschichtet.

2. Verfahren nach Anspruch 1, **dadurch gekennzeichnet, dass** man die erste Stufe gemäß Stufe 1 des Anspruchs 1 durchführt und dass man in einer zweiten Stufe die Kerne mit einer Menge des Überzugsmittels, ausgedrückt als Trockenmasse bezogen auf die Kerne, zwischen 10 und 30 Gew.-% durch Pulverisation, beschichtet.

3. Verfahren nach den Ansprüchen 1 und 2, **dadurch gekennzeichnet, dass** im Verlauf der ersten Stufe die Kerne des Granulats 90 bis 84 Gew.-% Wirkstoffe enthalten und dass die Menge des Überzugsmittels im Verlauf der zweiten Stufe zwischen 15 und 25 Gew.-% liegt.

4. Verfahren nach Anspruch 1, **dadurch gekennzeichnet, dass** die Wirkstoffe aus Arzneimitteln oder aus Aminosäuren, die für die Ernährung von Wiederkäuern bestimmt sind, ausgewählt werden.

5. Verfahren nach Anspruch 1 oder 2, **dadurch gekennzeichnet, dass** die Wirkstoffe aus Methionin und Lysin-HCl ausgewählt werden.

6. Verfahren nach einem der vorausgehenden Ansprüche, **dadurch gekennzeichnet, dass** man das schmelzbare Bindemittel aus Polyethylenwachsen, Paraffinen, ggf. hydrierten Ölen oder Fetten, Fettsäuren mit 10 bis 32 Kohlenstoffatomen, entsprechenden Estern und Alkoholen auswählt.

7. Verfahren nach Anspruch 4, **dadurch gekennzeichnet, dass** das schmelzbare Bindemittel Stearinsäure ist.

8. Verfahren nach einem der vorausgehenden Ansprüche, **dadurch gekennzeichnet, dass** man eine ausreichende Menge des Polymeren hinzufügt, um eine Masse zu erhalten, die eine korrekte Passage durch die Düse erlaubt und keine Phänomene der Exsudatbildung während der Extrusion hervorruft.

9. Verfahren nach Anspruch 6, **dadurch gekennzeichnet, dass** das Polymer aus den Styrolcopolymeren mit Vinylpyridin, den Alkylcellulosen, den Hydroxyalkylcellulosen, Polyvinylpyrrolidon, den Polyethylenen ausgewählt wird.

10. Verfahren nach Anspruch 7, **dadurch gekennzeichnet, dass** das Polymer ein Copolymeres auf Basis von Styrol und Vinylpyridin ist.

11. Verfahren nach Anspruch 7, **dadurch gekennzeichnet, dass** das Polymer Ethylcellulose ist.

12. Granulatkerne für Wiederkäuer, zusammengesetzt aus einem Matrizengemisch aus 95 bis 80 % eines oder mehrerer Wirkstoffs/Wirkstoffe, 5 bis 20 % eines schmelzbaren Bindemittels, das ein Polymeres enthält, hergestellt nach Stufe 1 des Anspruchs 1.

13. Kerne nach Anspruch 12, zusammengesetzt aus einem Matrizengemisch aus 90 bis 84 Gew.-% eines oder mehrerer Wirkstoffs/Wirkstoffe, 10 bis 16 Gew.-% eines schmelzbaren Bindemittels, das ein Polymeres enthält, hergestellt nach Stufe 1 des Anspruchs 1.

14. Wirkstoffkerne, die nach Stufe 1 des Anspruchs 1 hergestellt sind, zur weiteren Beschichtung mit einer Umhüllung mit einer Dichte von mehr als 1,2.

## Claims

1. Process for the preparation of granules of dietary or therapeutic active ingredients intended for ruminants, **characterized in that** in a first stage, the cores of the said granules are prepared by extrusion of a mixture containing from 95 to 80 % by weight of active ingredients in the presence of a meltable binding agent in a quantity such that it allows the passage of the mass through the die, and of a polymer, the said cores of granules having, after spheronization and sieving, a density greater than 1.2 g/ml and a granule distribution of between 200 and 4000 microns, and in a second stage, the said cores are coated by spraying a coating layer which is protective in relation to the rumen.

2. Preparation process according to Claim 1, **characterized in that** the first stage is performed according to stage 1 of Claim 1, and in a second stage, the said cores are coated with a quantity of coating, expressed as dry matter relative to the said cores, of between 10 and 30 % by weight, by spraying.

3. Process according to Claims 1 and 2, **characterized in that** during the first stage, the cores of the said granules contain 90 to 84 % by weight of active ingredients, and during the second stage, the quantity of coating is between 15 and 25 % by weight.

4. Process according to Claim 1, **characterized in that** the active ingredients are chosen from medicinal products or amino acids intended as ruminant feed.

5. Process according to Claim 1 or 2, **characterized in that** the active ingredients are chosen from methionine and lysine hydrochloride.

6. Process according to any one of the preceding claims, **characterized in that** the meltable binding agent is chosen from polyethylene waxes, paraffins, oils or fats, which are optionally hydrogenated, fatty acids having 10 to 32 carbon atoms, the corresponding esters and alcohols.

7. Process according to Claim 4, **characterized in that** the meltable agent is stearic acid.

8. Process according to any one of the preceding claims, **characterized in that** a sufficient quantity of polymer is added to obtain a mass allowing correct passage through the die and not causing exudation phenomena during extrusion.

9. Process according to Claim 6, **characterized in that** the polymer is chosen from the copolymers of styrene with vinylpyridines, alkylcelluloses, hydroxyalkylcelluloses, polyvinylpyrrolidones and polyethylenes.

10. Process according to Claim 7, **characterized in that** the polymer is a copolymer based on styrene and vinylpyridine.

11. Process according to Claim 7, **characterized in that** the polymer is ethylcellulose.

12. Cores of granules intended for ruminants, composed of a matrix mixture of 95 to 80 % of one or more active ingredients, of 5 to 20 % of a meltable binder and containing a polymer, which are prepared according to stage 1 of Claim 1.

13. Cores according to Claim 12, composed of a matrix mixture of 90 to 84 % by weight of one or more active ingredients, of 10 to 16 % by weight of a meltable binder and containing a polymer, which are prepared according to stage 1 of Claim 1.

14. Cores of active ingredients which are to be subsequently subjected to coating, having a density greater than 1.2, and which are prepared according to stage 1 of Claim 1.
